# EUROPEAN PATENT SPECIFICATION

(11) **EP 0 811 382 B1**
(45) Date of publication and mention of the grant of the patent: **06.09.2000**
(21) Application number: 96108873.9
(22) Date of filing: 03.06.1996
(51) Int. Cl.: A61K 38/08, A61K 38/09

(54) **Mammography method**
Mammographieverfahren
Procédé de mammographie

(43) Date of publication of application: 10.12.1997
(73) Proprietor: THE MEDICAL COLLEGE OF HAMPTON ROADS, Norfolk, VA 23507 (US)
(72) Inventor: Hodgen, Gary D., Eastern Virginia Medical School, Norfolk, VA 23507 (US)
(74) Representative: Vossius, Volker, Dr.

(56) References cited:
- AMERICAN JOURNAL OF ROENTGENOLOGY, vol. 157, no. 2, 1991, USA, pages 267-270, XP000600493 RUBENS ET AL: "GADOPENTETATE DIMEGLUMINE-ENHANCED CHEMICAL-SHIFT MR IMAGING OF THE BREAST"
- PHYSICS IN MEDICINE AND BIOLOGY, vol. 41, no. 3, 1996, BRISTOL, GB, pages 315-368, XP000580495 SAEBEL ET AL: "RECENT DEVELOPMENTS IN BREAST IMAGING"

## Description

There has been considerable debate about the value of mammograms for premenopausal women, particularly those whose age is between 35 and 50 years. It has been estimated that mammography misses about 40% of breast cancers in these premenopausal women because the threshold of sensitivity of mammography is decreased significantly by fibrous tissues and cystic ductal elements of the breasts. These dense tissue structures, along with edematous filling of stromal and ductal regions often cloud the radiologic film. In many younger women, a "negative" mammogram has meant a false sense of security about the absence of early breast disease and leads these women to be less vigilant about doing regular breast examinations.

It is known that the human breast undergoes profound histologic changes corresponding to the phases of the ovarian and menstrual cycles. See, e.g., Fanger et al., Cyclic Changes of Human Mammary Gland Epithelium in Relation to the Menstrual Cycle and Ultrastructural Study, Cancer 34:574-85 (1974); and Vogel et al., The Correlation of Histologic Changes in the Human Breast With the Menstrual Cycle, Mam J. Pathol. 1981 104:23-34.

Peck et al., Estrogen and Post-Menopausal Breast - Mammographic Considerations, Jama, 240:1733-35, 1978, reported on the effect of post-menopausal estrogen replacement therapy on mammographic studies. They noted that estrogen maintained the breast tissue, so that in the post-menopausal women the breast remained firm and the dense breast tissue could obscure a small cancer that otherwise could be palpitated or detected by mammography. Studies withdrawing the hormone replacement therapy for six months or more lead the authors to suggest that malignant neoplasm could be easier to demonstrate if the women of this high risk group did not receive exogenous estrogens.

In the Journal of the Canadian Association of Radiologists, 32:159, 1981, Ouimet-Oliva et al. described a study carried out with 25 women ranging in age from 22 to 47 years afflicted with severe mammary dysplasia. The women were given 400 milligrams per day of danazol (17-alpha-pregna-2,4-dien-20-yno(2,3-d)-isoxazol-17-ol), a synthetic androgen derived from ethisterone. After three months of treatment, the volume and density of the breast had decreased in 80% of the patients and at the end of the treatment, 23 of the 25 patients showed further regression in volume and especially in density such that the entire texture of the breast became exposed. However, danazol has many androgenic side effects. In addition to vasomotor flush of estrogen deprivation, it causes weight gain, long muscle cramps, breast atrophy, hot flashes, mood swings, oily skin, depression, edema, acne, fatigue, hirsutism, alterations in the libido, headache, rash, and a deepening of the voice. Therefore, while such a course of therapy may be tolerable for women afflicted with severe mammary dysplasia, typically it is not practical for other women, especially premenopausal women undergoing a once a year mammography screening.

It is accordingly the object of the present invention to provide a method for enhancing the mammographic films so as to facilitate the analysis of those films by the radiologist in their search for early identifications of either neoplasms or benign cysts. This and other objects of the invention would become apparent to those of ordinary skill in this from the following detailed description.

This invention relates to enhancing the readability of a mammogram and more particularly to enhancing the readability by administering to the premenopausal women prior to conducting the mammographic procedure a gonadotropin releasing hormone antagonist or mime thereof and then conducting the mammographic procedure before the effect of the gonadotropin releasing hormone antagonist has dissipated.

In accordance with the present invention, the conventional mammographic procedure is carried out but the readability of the radiologic film is enhanced by administering a gonadotropin releasing hormone antagonist or other inhibitor of ovarian steroidal supply impact on breast tissue ("mime") thereof to the women before that procedure is effected.

The gonadotropin releasing hormone is a small polypeptide produced in the hypothalamus and is sometimes termed gonadotropic releasing hormone, luteinizing hormone releasing hormone, GnRH or LHRH. Analogs in the form of antagonists and agonists are known. The present invention employs only the gonadotropin releasing hormone antagonist. A GnRH antagonist acts by classical competitive receptor occupancy at the level of the GnRH receptor in the anterior pituitary. The effect is realized quickly and the more active antagonists can extinguish bioactive gonadotropin secretion within minutes, and in turn deplete gonadal estrogen, progesterone and androgen synthesis and secretion to castrate levels within the first day of treatment without a "flare effect" (stimulation of the receptor system), and in turn, without a delay in therapeutic benefit and without a transient exacerbation by temporary elevations of estrogen and androgen.

Examples of gonadotropin releasing hormone antagonists can be found, iter alia, in United States Patents 4,409,208, 4,547,370, 4,565,804, 4,569,927, 4,619,914, and 5,198,533 and in WO 89/01944. Examples of such antagonists include Antide (a decapeptide represented by the formula D-Ac-D-2-Nal¹-DpClPhe²-D-3-Pal³-Ser⁴-NiLys⁵-D-NicLys⁶-Leu⁷-ILys⁸-Pro⁹-D-Ala¹⁰), [Ac-D4ClDPhe¹, D4ClDPhe², DTrp³, DArg⁶, DAla¹⁰] GnRH, [Ac-4ClDPhe², D₃Pal³, Arg⁵, D₂Nal⁶, DAla¹⁰] GnRH, [Ac-D2-Nal¹, 4ClDPhe², DTrp³, DArg⁶, DAla¹⁰] GnRH, [Ac-D₂Nal¹, 4FDPhe², DTrp³, DArg⁶] GnRH, [Ac-D2Nal¹, 4ClDPhe², DTrp³, DhArg(Et₂)⁶, DAla¹⁰] GnRH, and [Ac-Nal¹, DME4ClPhe², DPal³, Ser⁴, Tyr⁵, DArg⁶, Leu⁷, ILys⁸, Pro⁹, DAla¹⁰] GnRH.

Alternatively, a substitute for the gonadotropin releasing hormone antagonist can be employed. The inhibitors of steroid production or action are entities which mimic the activity of the antagonist sufficiently to reversibly inactivate gonadal response or impact of ovarian steroids on breast tissue, including the blockade of gonadotropin stimulated steroidogenesis. Examples include recombinant DNA derived gonadotropins, desialated gonadotropins whether natural or DNA derived, antibodies to gonadotropins, gonadotropic subunit parts, inhibitors of gonadotropin receptor activations (i.e., cell messengers), inhibin/activin and their analogs.

The gonadotropin releasing hormone antagonists or other inhibitors employed in the present invention can be administered in the form of pharmaceutically acceptable non-toxic salts or complexes. The salts include acid addition salts such as for instance hydrochloride, hydrobromide, sulfate, phosphate, nitrate, oxalate, fumarate, gluconate, tannate, maleate, acetate, benzoate, succinate, alginate, malate, ascorbate and tartrate. The complexes can be with metals such as for example zinc, barium, calcium, magnesium and aluminum.

The gonadotropin releasing hormone antagonist or other inhibitor administration aspect of the present invention is similar to the previous use of such antagonists for the treatment of endometriosis and/or uterine leiomyomata. Thus not only any known antagonist or inhibitor can be employed, but also the mode of administration heretofore employed can also be employed in the practice of the present invention. Thus, the route of administration can be any conventional route where the analog is active, for instance orally, intravenously, subcutaneously, intramuscularly, sublingually, percutaneously, rectally, intranasally or intravaginally. Similarly, the administration form can be a tablet, dragee, capsule, pill, nasal mist, aerosol, solutions suspensions or suppositories.

As a rule of thumb, the amount of gonadotropin releasing hormone antagonist or inhibitor administered is that sufficient to adjust the circulating estrogen to a value below 20 pg/ml and preferably below 10 pg/ml. Depending on the particular active agent employed, the dose administered is generally 0.001 to 5 mg/kg, preferably 0.05 to 5 mg/kg when administered intramuscularly. Also depending upon the particular agent employed, a single administration may suffice, although one or more additional administrations can be employed over a time period of about one week or up to 30 days, including daily, weekly or monthly. Since the effects of the administration last for several days, it is preferred that the or the initial gonadotropin releasing hormone antagonist or mime administration occur 7 to 10 days before the mammogram. The depletion of the circulating estrogen causes a transitory thinning of the breast tissue and possible relief of edema. Accordingly, the mammogram is carried out in conventional fashion before endogenous estrogen or progesterone production has been restored spontaneously and causes the breast to revert to its preadministration condition.

In order to demonstrate the present invention, women are first given a mammogram and thereafter administered the gonadotropin releasing hormone antagonist specified below intramuscularly or subcutaneously. One week later the mammogram is repeated resulting in a film which is clearer and easier to interpret in the diagnosis of breast cancer or benign breast lesions. The antagonist and amount which is administered after suspension in sesame oil are:

| Antagonist | Dose mg/kg/day |
|---|---|
| Antide | 0.3 |
| Azaline B | 0.05 |
| [Ac-D4ClDPhe¹, D4ClDPhe², DTrp³, DArg⁶, DAla¹⁰] GnRH | 0.5 |
| [Ac-4ClDPhe², D₃Pal³, Arg⁵, D₂Nal⁶, DAla¹⁰] GnRH | 0.5 |
| [Ac-D2-Nal¹, 4ClDPhe², DTrp³, DArg⁶, DAla¹⁰] GnRH | 0.5 |
| [Ac-Nal¹, DME4ClPhe², DPal³, Ser⁴, Tyr⁵, DArg⁶, Leu⁷, ILys⁸, Pro⁹, DAla¹⁰] GnRH | 0.5 |

Application of the components, compositions and methods of this invention for the medical and/or pharmaceutical use which are described in this text may be accomplished by any clinical, medical or pharmaceutical methods or techniques as are presently or prospectively known to those skilled in the art. The various embodiments which have been described herein were intended to be representative and not limiting, as various changes and modifications can be made in the present invention without departing from the spirit and scope thereof.

## Claims

1. A mammographic method in which the image on the mammographic film has enhanced readability, and in turn diagnostic utility, which comprising administering to a women an effective amount of a gonadotropin releasing hormone antagonist or mime thereof and effecting the mammogram before the effect of the antagonist or mime thereof has dissipated.

2. The method of claim 1 wherein the antagonist is administered.

3. The method of claim 2 wherein the antagonist is administered 7 to 30 days before the mammogram.

4. The method of claim 3 in which only a single administration of the antagonist is effected.

5. The method of claim 2 in which only a single administration of the antagonist is effected.

6. The method of claim 2 in which 0.001 to 5 mg/kg of antagonist is administrated.

7. The method of claim 2 in which 0.5 to 5 mg/kg of antagonist is administrated.

8. The method of claim 2 in which the antagonist is antide.

9. The method of claim 2 in which the antagonist is azaline B.

10. The method of claim 1 wherein the antagonist or mime is administered 7 to 30 days before the mammogram.

11. The method of claim 10 in which only a single administration of the antagonist is effected.

12. The method of claim 1 in which only a single administration of the antagonist or mime is effected.

13. The method of claim 1 in which 0.001 to 5 mg/kg of antagonist or mime is administrated.

14. The method of claim 1 in which 0.5 to 5 mg/kg of antagonist or mime is administrated.

15. The use of a gonadotropin releasing hormone antagonist or mime thereof for enhancing readability of the image on a mammographic film after administration to a premenopausal woman according to any one of claims 1 to 14.

## Patentansprüche

1. Mammographieverfahren, wobei das Bild auf dem mammographischen Film eine verbesserte Auswertbarkeit und daher diagnostischen Nutzen aufweist, umfassend das Verabreichen einer wirksamen Menge eines Gonadotropin-freisetzenden Hormonantagonisten oder einer die Aktivität des Antagonisten nachahmenden Substanz an eine Frau und Aufnehmen des Mammogramms, bevor die Wirkung des Antagonisten oder der die Aktivität des Antagonisten nachahmenden Substanz erloschen ist.

2. Verfahren gemäß Anspruch 1, wobei der Antagonist verabreicht wird.

3. Verfahren gemäß Anspruch 2, wobei der Antagonist 7 bis 30 Tage vor dem Mammogramm verabreicht wird.

4. Verfahren gemäß Anspruch 3, wobei nur eine einzige Verabreichung des Antagonisten erfolgt.

5. Verfahren gemäß Anspruch 2, wobei nur eine einzige Verabreichung des Antagonisten erfolgt.

6. Verfahren gemäß Anspruch 2, wobei 0.001 bis 5 mg/kg des Antagonisten verabreicht werden.

7. Verfahren gemäß Anspruch 2, wobei 0.5 bis 5 mg/kg des Antagonisten verabreicht werden.

8. Verfahren gemäß Anspruch 2, wobei der Antagonist Antide ist.

9. Verfahren gemäß Anspruch 2, wobei der Antagonist Azalin B ist.

10. Verfahren gemäß Anspruch 1, wobei der Antagonist oder die die Aktivität des Antagonisten nachahmende Substanz 7 bis 30 Tage vor dem Mammogramm verabreicht wird.

11. Verfahren gemäß Anpruch 10, wobei nur eine einzige Verabreichung des Antagonisten erfolgt.

12. Verfahren gemäß Anspruch 1, wobei nur eine einzige Verabreichung des Antagonisten oder der die Aktivität des Antagonisten nachahmenden Substanz erfolgt.

13. Verfahren gemäß Anspruch 1, wobei 0.001 bis 5 mg/kg des Antagonisten oder der die Aktivität des Antagonisten nachahmenden Substanz verabreicht werden.

14. Verfahren gemäß Anspruch 1, wobei 0.5 bis 5 mg/kg des Antagonisten oder der die Aktivität des Antagonisten nachahmenden Substanz verabreicht werden.

15. Verwendung eines Gonadotropin-freisetzenden Hormonantagonisten oder einer die Aktivität des Antagonisten nachahmenden Substanz zur Verbesserung der Auswertbarkeit des Bilds eines mammographischen Films nach dem Verabreichen an eine Frau in der Prämenopause gemäß einem der Ansprüche 1 bis 14.

## Revendications

1. Procédé mammographique selon lequel l'image présente sur le film mammographique possède une facilité de lecture accrue, et par suite une utilité de diagnostic, qui consiste à administrer à une femme une quantité efficace d'un antagoniste de l'hormone de libération de gonadotrophine ou un agent imitant celui-ci et à effectuer la mammographie avant que l'effet de l'antagoniste ou de l'agent imitant celui-ci se soient dissipés.

2. Procédé de la revendication 1, selon lequel on administre l'antagoniste.

3. Procédé de la revendication 2, selon lequel on administre l'antagoniste 7 à 30 jours avant la mammographie.

4. Procédé de la revendication 3, selon lequel on n'effectue qu'une administration unique de l'antagoniste.

5. Procédé de la revendication 2, selon lequel on n'effectue qu'une administration unique de l'antagoniste.

6. Procédé de la revendication 2, selon lequel on administre 0,001 à 5 mg/kg d'antagoniste.

7. Procédé de la revendication 2, selon lequel on administre 0,5 à 5 mg/kg d'antagoniste.

8. Procédé de la revendication 2, selon lequel l'antagoniste est l'antide.

9. Procédé de la revendication 2, selon lequel l'antagoniste est l'azaline B.

10. Procédé de la revendication 1, selon lequel on administre l'antagoniste ou l'agent imitant celui-ci 7 à 30 jours avant la mammographie.

11. Procédé de la revendication 10, selon lequel on n'effectue qu'une administration unique de l'antagoniste.

12. Procédé de la revendication 1, selon lequel on n'effectue qu'une administration unique de l'antagoniste ou agent imitant celui-ci.

13. Procédé de la revendication 1, selon lequel on administre 0,001 à 5 mg/kg d'antagonistes ou d'agent imitant celui-ci.

14. Procédé de la revendication 1, selon lequel on administre 0,5 à 5 mg/kg d'antagoniste ou d'agent imitant celui-ci.

15. Utilisation d'un antagoniste de l'hormone de libération de gonadotrophine ou d'un agent imitant celui-ci pour accroître la facilité de lecture de l'image présente sur un film mammographique après administration à une femme préménopausée conformément à l'une quelconque des revendications 1 à 14.
